# EUROPÄISCHE PATENTSCHRIFT

(11) **EP 0 869 936 B1**
(45) Veröffentlichungstag und Bekanntmachung des Hinweises auf die Patenterteilung: **16.08.2000**
(21) Anmeldenummer: 96904777.8
(22) Anmeldetag: 14.02.1996
(51) Int. Cl.: C07C 29/88, C07C 31/02

(54) **VERFAHREN ZUR DESTILLATION VON ALKOHOLEN**
METHOD FOR DISTILLING ALCOHOLS
PROCEDE POUR DISTILLER DES ALCOOLS

(30) Priorität: 23.02.1995 DE 19506280
(43) Veröffentlichungstag der Anmeldung: 14.10.1998
(73) Patentinhaber: Celanese Chemicals Europe GmbH, 60439 Frankfurt am Main (DE)
(72) Erfinder: ZGORZELSKI, Wolfgang, D-46149 Oberhausen (DE); LAPPE, Peter, D-46539 Dinslaken (DE); SCHALAPSKI, Kurt, D-46147 Oberhausen (DE); GICK, Wilhelm, D-47199 Duisburg (DE)
(86) Internationale Anmeldenummer: EP9600633
(87) Internationale Veröffentlichungsnummer: WO9626173

(56) Entgegenhaltungen:
- US-A- 2 533 754
- US-A- 2 626 284
- US-A- 2 753 297
- US-A- 2 889 375
- US-A- 3 359 335
- US-A- 3 576 891
- US-A- 3 689 371
- US-A- 3 960 672

## Beschreibung

Aliphatische C₃-C₁₀-Alkohole, wie n-Butanol und insbesondere 2-Ethylhexanol besitzen eine hohe wirtschaftliche Bedeutung. Die Herstellung dieser Alkohole erfolgt bevorzugt durch Hydroformylierung von Olefinen mit nachfolgender Hydrierung der intermediär gebildeten Aldehyde (Beispiel: Propylen → n/i-Butyraldehyd → n/i-Butanole) oder durch Aldolisierung von geradkettigen aliphatischen Aldehyden zu den entsprechenden ungesättigten Aldehyden und nachfolgende Hydrierung (Beispiel: n-Butyraldehyd → 2-Ethylhexenal → 2-Ethylhexanol). Zusammenfassende Darstellungen finden sich z.B. in Ullmann's Encyclopedia of Industrial Chemistry: "Alcohols, Aliphatic" (Vol. A 1), "2-Ethylhexanol" (Vol. A 10) und "Butanols" (Vol. A 4).

Außer als Lösungsmittel wird n-Butanol vor allem auf dem Farben- und Lacksektor und zur Herstellung von Carbonsäureestern, insbesondere n-Butylacrylat und Di-n-butylphthalat (DBP) verwendet.

2-Ethylhexanol wird vornehmlich als Alkoholkomponente zur Herstellung von Di-2-ethylhexylphthalat (DEHP) und 2-Ethylhexylacrylat benötigt.

Für diese Anwendungsbereiche - insbesondere die Herstellung von Acrylsäureester - ist der Einsatz hochreiner Alkohole zwingend notwendig. Bei der technischen Herstellung der Alkohole erfolgt die Reinigung durchwegs durch mehrstufige fraktionierte Destillation. Die Alkohole werden hierbei über einen Zeitraum von mehreren Stunden einer thermischen Belastung ausgesetzt, wobei in der Regel Sumpftemperaturen von 150 bis 200°C angewandt werden. Als Folge hiervon kommt es bei der Destillation von aliphatischen C₃-C₁₀-Alkoholen wie Butanol und 2-Ethylhexanol zur Bildung der entsprechenden Aldehyde, die unter üblichen, in der Technik angewandten Bedingungen, nur mit hohem Aufwand abgetrennt werden können.

Ferner sind aus dem Stand der Technik verschiedene destillative Reinigungsverfahren von Alkoholen unter Zusatz von basischen Verbindungen bekannt.

So betrifft US-A-2,533,754 ein Verfahren zur Reinigung eines aldehydhaltigen Ethanols unter Zusatz von Alkalimetallhydroxiden in einer Menge von 0,1 bis 3 Gew.-%, bezogen auf den zu reinigenden Alkohol. Gemäß einer beispielhaften Ausführung wird einer 88,3 Gew.-%igen Lösung an Ethanol Natriumhydroxid in einer solchen Menge zugesetzt, daß der Natriumhydroxidgehalt in der Lösung 1,46 Gew.-% beträgt.

Aus US-A-2,889,375 ist ein Verfahren zur destillativen Reinigung von Oxoalkoholen bekannt, die geringe Mengen an Aldehydverbindungen als Verunreinigungen enthalten. Das offenbarte Verfahren ist dadurch charakterisiert, daß dem Kolonnensumpf Erdalkalimetallverbindungen, insbesondere Oxide, Hydroxide oder Carbonate in einer Menge von 1 Gew.-%, bezogen auf den Einsatz, zugesetzt werden. Nach der Lehre von US-A-2,889,375 führt jedoch der Zusatz von Alkaliverbindungen, wie Natriumcarbonat oder Natriumhydroxid während der Destillation zu erheblichen Alkoholverlusten infolge der Bildung von Kondensationsprodukten.

US-A-3,689,371 offenbart ein Verfahren zur destillativen Reinigung von Butanolen aus der Oxosynthese. Der anfallende Rohalkohol wird zunähst mit einer wäßrigen Alkalimetallhydroxidlösung behandelt, um die im Rohalkohol enthaltenen Säuren und Ester zu neutralisieren bzw. zu spalten. Nach dem bekannten Verfahren werden zunächst die Alkalihydroxide, beispielsweise durch eine Wasserwäsche, aus dem Rohalkohol entfernt. Anschließend wird der alkalihydroxidfreie Rohalkohol in die weitere destillative Aufarbeitung gefahren.

Nach US-A-3,960,672 lassen sich niedrige C₁-C₃-Alkohole mittels einer zweistufigen Extraktionsdestillation reinigen, indem eine wäßrige Alkalilösung auf den oberen Boden der Kolonne aufgegeben wird. Das Verfahren der Extraktivdestillation, das mit großen Mengen Wasser arbeitet, wird gemäß der Offenbarung von US-A-3,960,672 zur Reinigung niedriger Alkohole durchgeführt, die sich mit Wasser zu einer homogenen Flüssigkeit verdünnen lassen.

Es bestand daher die Aufgabe, ein einfaches Verfahren zur Destillation von C₃-C₁₀-Alkoholen zu finden, das diese Nachteile nicht aufweist.

Überraschenderweise wurde gefunden, daß durch Zugabe von geringen Mengen von Alkalihydroxid die Bildung der entsprechenden Aldehyde während der destillativen Aufarbeitung unterdrückt wird und sogar bereits vorhandene Aldehyde beseitigt werden.

Gegenstand der vorliegenden Erfindung ist daher ein Verfahren zur destillativen Reinigung von C₃-C₁₀-Alkoholen, dadurch gekennzeichnet, daß man die Alkohole bei 150 bis 200°C in Gegenwart von 10 bis 1000 ppm Alkalihydroxid destilliert, wobei eine Extraktivdestillation ausgeschlossen ist.

Die C₃-C₁₀-Alkohole können geradkettig oder verzweigt sein. Besonders wichtige C₃-C₁₀-Alkohole, bei denen das erfindungsgemäße Verfahren anwendbar ist, sind n-Butanol und 2-Ethylhexanol.

Als Alkalihydroxide werden vorzugsweise KOH oder NaOH eingesetzt. Die Menge an Alkalihydroxid beträgt im allgemeinen 10 bis 1000 ppm, vorzugsweise 10 bis 200 ppm, jeweils bezogen auf die Menge des eingesetzten Alkohols. Vorzugsweise werden die Alkalihydroxide in Form einer wäßrigen Lösung zugegeben.

Falls bei der Destillation Kolonnen eingesetzt werden, so haben diese im allgemeinen 20 bis 80 Böden.

Wie die folgenden Beispiele zeigen, wird durch die Zugabe von Alkalihydroxid die Bildung von Aldehyden während der Destillation der Alkohole stark vermindert oder sogar ganz verhindert, ohne daß es zu anderen unerwünschten Nebenreaktionen oder zu wesentlicher Verminderung des Alkoholgehaltes kommt. Häufig wird sogar ein bereits im eingesetzten Alkohol enthaltener Aldehydanteil beseitigt.

In den Beispielen bezieht sich die Angabe "Leichtsieder" auf Komponenten, die frei sind von 2-Ethylhexanal und wesentlich tiefer sieden (mindestens 20 bis 40°C tiefer) als 2-Ethylhexanol und daher leicht von diesem destillativ zu trennen sind. Analog bezieht sich die Angabe "Hochsieder" auf Komponenten, die frei sind von 2-Ethylhexanal und wesentlich höher sieden (mindestens 20 bis 40°C höher) als 2-Ethylhexanol und daher ebenfalls leicht von diesem destillativ zu trennen sind.

### Vergleichsbeispiel 1

2-Ethylhexanol mit einem Gehalt von 60 ppm (0.006 Gew.-%) 2-Ethylhexanal wurde ohne Zusatz von Alkalihydroxid 1 Stunde lang auf 180°C erhitzt. Nach 10, 30 und 60 Minuten wurde jeweils eine Probe entnommen und analysiert. Die Ergebnisse sind in Tabelle 1 angegeben. Nach 60 Minuten betrug der Gehalt an 2-Ethylhexanal 140 ppm (0.014 Gew.-%).

### Beispiel 1

Es wurde vorgegangen wie in Vergleichsbeispiel 1, mit der einzigen Ausnahme, daß vor Beginn der Erhitzung dem 2-Ethylhexanol 50 ppm KOH zugesetzt wurden. Die Ergebnisse sind wieder in Tabelle 1 angegeben. Nach 30 Minuten lag der Gehalt an 2-Ethylhexanal bereits unterhalb der analytischen Nachweisgrenze von 0.001 Gew.-%.

**Tabelle 1**

| | Ausgangsmaterial vor Beginn | Vergleichsbeispiel (ohne Alkalihydroxid) Zeit seit Beginn | | | Beispiel 1 (50 ppm KOH) Zeit seit Beginn | | |
|---|---|---|---|---|---|---|---|
| | | 10 Min. | 30 Min. | 60 Min. | 10 Min. | 30 Min. | 60 Min. |
| Leichtsieder (Gew.%) | 0.216 | 0.212 | 0.211 | 0.212 | 0.208 | 0.209 | 0.207 |
| 2-Ethylhexanal (Gew.-%) | 0.006 | 0.011 | 0.013 | 0.014 | 0.002 | <0.001 | <0.001 |
| 2-Ethylhexanol (Gew.-%) | 99.646 | 99.644 | 99.643 | 99.640 | 99.616 | 99.616 | 99.616 |
| Hochsieder (Gew.-%) | 0.132 | 0.133 | 0.133 | 0.134 | 0.174 | 0.175 | 0.177 |

### Vergleichsbeispiel 2

In einer Labor-Füllkörperkolonne mit einer 1 m hohen Schüttung von 3 mm VA-Wendeln werden 663 g 2-Ethylhexanol folgender Zusammensetzung (in Gew.-%) in einer 2 l Sumpfblase eingesetzt:

| | |
|---|---|
| Leichtsieder | 0.099 |
| 2-Ethylhexanal | 0.001 |
| 2-Ethylhexanol | 99.770 |
| Hochsieder | 0.130 |

Bei einem Kolonnendruck von 100 mbar und einem Rücklaufverhältnis von 3:1 wurden am Kolonnenkopf bei einer Temperatur von 118°C vier Destillationsfraktionen mit jeweils 130 g und eine 5. Fraktion mit 65 g abgenommen. Die einzelnen Fraktionen wurden gaschromatographisch untersucht:

**Tabelle 2**

| Fraktion | 1 | 2 | 3 | 4 | 5 |
|---|---|---|---|---|---|
| Leichtsieder (Gew.-%) | 0.010 | 0.006 | 0.003 | 0.011 | 0.008 |
| 2-Ethylhexanal (Gew.-%) | 0.013 | 0.009 | 0.008 | 0.010 | 0.011 |
| 2-Ethylhexanol (Gew.-%) | 99.863 | 99.980 | 99.986 | 99.975 | 99.977 |
| Hochsieder (Gew.-%) | 0.114 | 0.005 | 0.003 | 0.004 | 0.004 |

Im Destillationsrückstand (78 g) lag der Gehalt an 2-Ethylhexanal unterhalb der analytischen Nachweisgrenze.

### Beispiel 2

Es wurde gearbeitet wie in Vergleichsbeispiel 2, mit der einzigen Ausnahme, daß dem 2-Ethylhexanol vor Beginn der Destillation 66 mg einer 50 %igen wäßrigen KOH-Lösung zugesetzt werden. Die einzelnen Fraktionen wurden wieder gaschromatographisch untersucht:

**Tabelle 3**

| Fraktion | 1 | 2 | 3 | 4 | 5 |
|---|---|---|---|---|---|
| Leichtsieder (Gew.-%) | 0.010 | 0.006 | 0.003 | 0.011 | 0.008 |
| 2-Ethylhexanal (Gew.-%) | 0.003 | 0.002 | 0.001 | 0.001 | 0.001 |
| 2-Ethylhexanol (Gew.-%) | 99.863 | 99.987 | 99.993 | 99.984 | 99.987 |
| Hochsieder (Gew.-%) | 0.124 | 0.005 | 0.003 | 0.004 | 0.004 |

Im Destillationsrückstand (78 g) lag der Gehalt an 2-Ethylhexanal unterhalb der analytischen Nachweisgrenze.

## Patentansprüche

1. Verfahren zur destillativen Reinigung von C₃-C₁₀-Alkoholen, dadurch gekennzeichnet, daß man die Alkohole bei 150 bis 200°C in Gegenwart von 10 bis 1000 ppm Alkalihydroxid destilliert, wobei eine Extraktivdestillation ausgeschlossen ist.

2. Verfahren nach Anspruch 1, dadurch gekennzeichnet, daß man als C₃-C₁₀-Alkohole n-Butanol oder 2-Ethylhexanol einsetzt.

3. Verfahren nach Anspruch 1 oder 2, dadurch gekennzeichnet, daß man die Alkohole in Gegenwart von 10 bis 200 ppm Alkalihydroxid destilliert.

## Claims

1. A process for the purification of C₃-C₁₀-alcohols by distillation, which comprises distilling the alcohols at 150 to 200°C in the presence of 10 to 1000 ppm of alkali metal hydroxide, extractive distillation being ruled out.

2. The process as claimed in claim 1, wherein, as C₃-C₁₀-alcohols, use is made of n-butanol or 2-ethylhexanol.

3. The process as claimed in claim 1 or 2, wherein the alcohols are distilled in the presence of 10 to 200 ppm of alkali metal hydroxide.

## Revendications

1. Procédé pour la purification par distillation d'alcools en C₃-C₁₀, caractérisé en ce que l'on distille les alcools à de 150 à 200°C en présence de 10 à 1 000 ppm d'hydroxyde alcalin, une distillation extractive étant exclue.

2. Procédé selon la revendication 1, caractérisé en ce que l'on utilise comme alcools en C₃-C₁₀ du n-butanol ou du 2-éthylhexanol.

3. Procédé selon la revendication 1 ou 2, caractérisé en ce que l'on distille les alcools en présence de 10 à 200 ppm d'hydroxyde alcalin.
